# EUROPEAN PATENT APPLICATION

(11) **EP 4 696 470 A1**
(43) Date of publication of application: **18.02.2026**
(21) Application number: 24810237.8
(22) Date of filing: 11.05.2024
(51) Int. Cl.: B25J 19/00

(54) **FORCE BALANCING APPARATUS AND FORCE BALANCING SYSTEM**

(30) Priority: 19.05.2023 CN 202310576384
(71) Applicant: Cornerstone Technology (Shenzhen) Limited, Shenzhen, Guandong 518066 (CN)
(72) Inventor: ZHAO, Jinbao, Shenzhen, Guangdong 518066 (CN); WANG, Zerui, Shenzhen, Guangdong 518066 (CN); HUANG, Wenjie, Shenzhen, Guangdong 518066 (CN); LIU, Yu, Shenzhen, Guangdong 518066 (CN)
(74) Representative: Valet Patent Services Limited
(86) International application number: PCT/CN2024/092653
(87) International publication number: WO 2024/239990

(57) **Abstract**

A counterbalance device for an apparatus with composite joints is provided. The apparatus includes a frame, a first movable member supported by the frame, and a second movable member connected to the first movable member. The first movable member is translatable relative to the frame along a first line, and the second movable member is rotatable relative to the first movable member about a first axis perpendicular to the first line and travers to the direction of the force. The counterbalance device includes: a force output mechanism connected to the frame and configured to provide an output force; a distribution mechanism connected to the force output mechanism, to allow the force output mechanism to apply the output force to the distribution mechanism at a first load-bearing position of the distribution mechanism; and a pulling mechanism connected to the distribution mechanism, to allow the pulling mechanism to act on the distribution mechanism at a second load-bearing position of the distribution mechanism, the pulling mechanism being connected to the first movable member and translatable relative to the first movable member along the first line, the pulling mechanism being further connected to the second movable member, and the pulling mechanism being translatable along the first line with rotation of the second movable member about the first axis.

## Description

The present disclosure claims the benefits of and priority to Chinese Patent Application No. 202310576384.0, filed on May 19, 2023, the entire disclosure of which is hereby incorporated by reference, in its entirety, for all that it teaches and for all purposes.

### TECHNICAL FIELD

The present disclosure relates to the technical field of counterbalance devices, and in particular, to a counterbalance device and a counterbalance system.

### BACKGROUND

In some equipment (such as industrial robots, surgical robots, etc.), various joints are typically incorporated to provide one or more degrees of freedom for the end effector, such as translation, rotation, or the like. For instance, a robotic arm generally includes several links, where two adjacent links may be connected via a revolute joint to allow relative rotation and/or via a prismatic joint to enable relative translation. This configuration provides the distal end of the robotic arm with one or more degrees of freedom to execute predetermined operations.

In certain scenarios, the moving parts of these joints may be subjected to continuous external forces. For example, when the motion of a moving part resulting from the joints is in the vertical direction, or one of components of the motion is in the vertical direction, the movement of the joints is significantly affected by gravity. To facilitate more effortless and smooth movement (whether manual or motor-driven) of the joints, it is advantageous to partially or fully counterbalance such force.

### SUMMARY

Embodiments of the present disclosure provide a counterbalance device and a counterbalance system, which can streamline system design and contribute to reducing both the weight and size of the system.

According to a first aspect, the embodiments of the present disclosure provide a counterbalance device for an apparatus with composite joints. The apparatus includes a frame, a first movable member supported by the frame, and a second movable member connected to the first movable member. The first movable member is translatable relative to the frame along a first line, and the second movable member is rotatable relative to the first movable member about a first axis perpendicular to the first line. The counterbalance device includes: a force output mechanism connected to the frame and configured to provide an output force; a distribution mechanism connected to the force output mechanism, to allow the force output mechanism to apply the output force to the distribution mechanism at a first load-bearing position of the distribution mechanism; and a pulling mechanism connected to the distribution mechanism, to allow the pulling mechanism to act on the distribution mechanism at a second load-bearing position of the distribution mechanism, the pulling mechanism being connected to the first movable member and translatable relative to the first movable member along the first line, the pulling mechanism being further connected to the second movable member, and the pulling mechanism being translatable along the first line with rotation of the second movable member about the first axis.

According to a second aspect, the embodiments of the present disclosure provide a counterbalance device for counterbalancing an eccentric mass on a rotating arm supported by a frame, the rotating arm being rotatable relative to the frame about a first axis, and the counterbalance device including: a force output mechanism connected to the frame and configured to provide an output force; and a pulling mechanism connected to an output end of the force output mechanism, to allow the force output mechanism to apply the output force to the pulling mechanism, the pulling mechanism being connected to the frame and translatable relative to the frame along a first line perpendicular to the first axis, the pulling mechanism being further connected to the rotating arm, and the pulling mechanism being translatable along the first line with rotation of the rotating arm about the first axis.

According to a third aspect, the embodiments of the present disclosure provide a counterbalance system, including: a frame; a first movable member supported by the frame and translatable relative to the frame along a first line; a second movable member connected to the first movable member and rotatable relative to the first movable member about a first axis; the counterbalance device of the first aspect; and a connecting component connecting the second movable member and the pulling mechanism of the counterbalance device.

According to a fourth aspect, the embodiments of the present disclosure provide a counterbalance system, including: a frame; a rotating arm supported by the frame and rotatable relative to the frame about a first axis; the counterbalance device of the second aspect; and a connecting component connecting the rotating arm and the pulling mechanism of the counterbalance device.

According to a fifth aspect, the embodiments of the present disclosure provide a robotic arm connected to a frame. The robotic arm includes a first link translatable relative to the frame along a first line; a revolute joint connected to the first link, the revolute joint including a rotating member rotatable relative to the first link about a first axis perpendicular to the first line; and the counterbalance device of the first aspect.

According to a sixth aspect, the embodiments of the present disclosure provide a robotic arm connected to a frame. The robotic arm includes a revolute joint connected to the frame, the revolute joint including a rotating member rotatable relative to the frame about a first axis; and the counterbalance device of the second aspect.

According to a seventh aspect, the embodiments of the present disclosure provide a medical system, including the robotic arm of the fifth aspect, and/or the robotic arm of the sixth aspect.

### BRIEF DESCRIPTION OF THE DRAWINGS

In order to more clearly illustrate the technical solutions of the embodiments of the present disclosure, the accompanying drawings to be used in the embodiments will be briefly described below. For those skilled in the art, other drawings based on these provided ones may be obtained without creative effort.
FIG. 1 is a schematic structural diagram of a robotic arm of an apparatus according to an embodiment of the present disclosure.
FIG. 2 is a schematic diagram of a counterbalance device for a revolute joint according to an embodiment of the present disclosure.
FIG. 3A is a schematic diagram of a counterbalance device for a revolute joint according to another embodiment of the present disclosure.
FIG. 3B shows a variant of the embodiment in FIG. 3A.
FIG. 4 is a schematic diagram of a counterbalance device for a revolute joint according to yet another embodiment of the present disclosure.
FIG. 5 is a schematic diagram of a counterbalance device for composite joints according to an embodiment of the present disclosure.
FIG. 6 is a schematic diagram of a counterbalance device for composite joints according to another embodiment of the present disclosure.
FIG. 7 is a schematic diagram of a counterbalance device for composite joints according to yet another embodiment of the present disclosure.
FIG. 8 is a schematic structural diagram of a slave operation apparatus of a medical system according to an embodiment of the present disclosure.
FIG. 9 is a partial structural diagram of a robotic arm of a slave operation apparatus according to an embodiment of the present disclosure.
FIG. 10 is another partial structural diagram of a robotic arm of a slave operation apparatus according to an embodiment of the present disclosure.
FIG. 11 is a partial exploded structural diagram of a moment arm adjustment mechanism of a robotic arm in a slave operation apparatus according to an embodiment of the present disclosure.
FIG. 12 is a schematic structural diagram of a moment arm adjustment mechanism of a robotic arm in a slave operation apparatus according to an embodiment of the present disclosure.
FIG. 13 is a schematic structural diagram of a force output mechanism of a robotic arm in a slave operation apparatus according to an embodiment of the present disclosure.
FIG. 14 is a schematic structural diagram of a distribution mechanism in a robotic arm of a slave operation apparatus according to a first embodiment of the present disclosure.
FIG. 15 is a schematic structural diagram of the distribution mechanism and part of its connected components in FIG. 14.
FIG. 16 is a schematic structural diagram showing another perspective of the distribution mechanism and part of its connected components in FIG. 15.
FIG. 17 is another schematic structural diagram of the distribution mechanism and part of its connected components in FIG. 15.
FIG. 18 is a schematic structural diagram of a distribution mechanism in a robotic arm of a slave operation apparatus according to a second embodiment of the present disclosure.
FIG. 19 is a schematic structural diagram of the distribution mechanism and part of its connected components in FIG. 18.
FIG. 20 is a schematic structural diagram of a distribution mechanism in a robotic arm of a slave operation apparatus according to a third embodiment of the present disclosure.
FIG. 21 is a schematic structural diagram of the distribution mechanism and part of its connected components in FIG. 20.

### DETAILED DESCRIPTION OF THE EMBODIMENTS

In order to make the objectives, technical solutions, and advantages of the embodiments of the present disclosure clearer, the embodiments of the present disclosure will be described in detail below with reference to the accompanying drawings. However, one skilled in the art can understand that in the embodiments of the present disclosure, technical details are set forth to provide a better understanding of the present disclosure. Nevertheless, the technical solutions sought to be protected by the present disclosure may be implemented even without these technical details and without various changes and modifications based on the following embodiments.

In the embodiments of the present disclosure, the terms "upper", "lower", "left", "right", "front", "rear", "top", "bottom", "inner", "outer", "central", "vertical", "horizontal", "transverse", and "longitudinal", as well as other terms indicating orientation or positional relationships, are based on the orientation or positional relationships shown in the accompanying drawings. These terms are used to facilitate the description of the present disclosure and the embodiments, and are not intended to limit the specific orientation of the devices, elements, and components, or to limit the specific operating orientation for the devices, elements, and components.

In addition to indicating orientation or positional relationships, the aforementioned terms may also denote other meanings. For example, the term "upper" may, in certain circumstances, denote an attachment relationship or connection relationship. It should be understood by one skilled in the art that the specific meanings of these terms in the present disclosure may be interpreted depending on the specific context.

Further, the terms "mounted", "arranged", "provided with", "defined with", "connected", and "coupled" are to be construed broadly. For example, a connection may be a fixed connection, a detachable connection, or an integral construction; may be a mechanical connection or an electrical connection; or may be a direct connection, an indirect connection via an intermediate medium, or internal communication between two devices, elements, or components. One skilled in the art can understand that the specific meanings of these terms in the present disclosure may be interpreted depending on the specific circumstances.

It should be noted that herein, relational terms such as "first" and "second" are used solely to distinguish one part or operation from another part or operation, without necessarily requiring or implying any actual relationship or order between such parts or operations. Further, the terms "include", 'have", or any other variations thereof are intended to cover a non-exclusive inclusion, such that a process, method, article, or apparatus including a list of elements includes not only those elements, but also includes other elements not expressly listed, or further includes elements inherent to such process, method, article, or apparatus. In the absence of additional constraints, an element defined by the phrase "including ..." does not preclude the existence of additional identical elements in the process, method, article, or apparatus that includes the element.

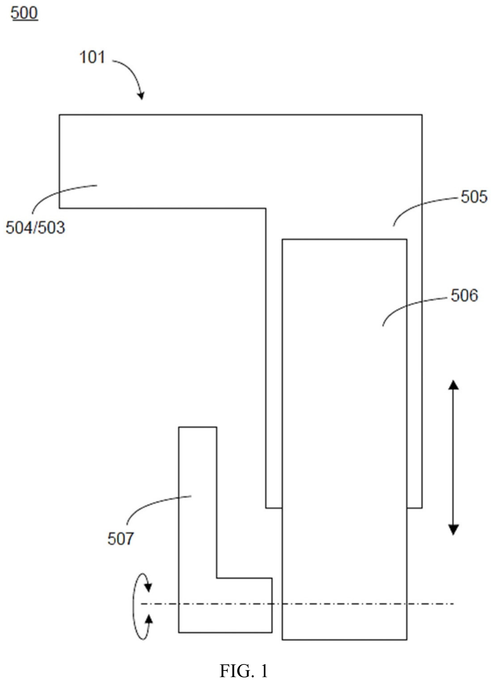

In some apparatus (such as industrial robots, surgical robots), various joints are typically incorporated to provide one or more degrees of freedom for the end effector, such as translation, rotation, or the like. For instance, a robotic arm generally includes several links, where two adjacent links may be connected via a revolute joint to allow relative rotation and/or via a prismatic joint to enable relative translation. This configuration provides the distal end of the robotic arm with one or more degrees of freedom to execute predetermined operations. For example, referring to FIG. 1, a robotic arm 101 includes at least three links, e.g., a link 505, a link 506, and a link 507. The link 505 and the link 506 are connected via a prismatic joint, while the link 506 and the link 507 are connected via a revolute joint. The link 505 is connected to a frame (not shown), the link 506 is translatable relative to the link 505 along a predetermined path, and the link 507 is rotatable relative to the link 506 about a predetermined axis. The link 507 may be connected with an end effector. Alternatively, the link 507 may be connected to a further link via a further joint. The frame may be either a part of the apparatus that is stationary relative to the ground, or a part of the apparatus that is movable relative to the ground, which is not limited herein.

In certain scenarios, the moving parts of these joints may be subjected to continuous external forces. For example, when the motion of a moving part resulting from the joints is in the vertical direction, or one of components of the motion is in the vertical direction, the movement of the joints is significantly affected by gravity. To facilitate more effortless and smooth movement (whether manual or motor-driven) of the joints, it is advantageous to partially or fully counterbalance such force.

### Counterbalance device for Revolute joint

In certain scenarios, a rotating arm (e.g., the link 507 in FIG. 1) is supported by a frame of an apparatus via at least a revolute joint. The rotating arm and/or members connected thereto are subjected to a continuous force. The revolute joint is rotatable about an axis transverse to the direction of the force applied, and the point of force application is offset from the axis of the revolute joint. In some embodiments, the axis of the revolute joint is perpendicular to the direction of the force. As used herein, the term "transverse to" denotes a non-parallel relationship, including, but not limited to, configurations where two members intersect at an angle or are skew to each other. For example, when the axis of the revolute joint is transverse to the direction of gravity, the aforementioned force may include the gravitational force. Due to the gravitational force acting on the rotating arm and other members, it is necessary to partially or fully counterbalance the gravity-induced torque (hereinafter referred to as gravitational torque) relative to the axis. This makes rotation of the rotating arm more effortless and smoother, while reducing the impact of the rotating arm on the revolute joint. Under conditions of complete counterbalance, the rotating arm may even be brought into a state of neutral equilibrium.

Currently, the aforementioned counterbalance may be implemented through active counterbalance approaches by using powered members such as motors or pneumatic actuators, or passive counterbalance approaches by using counterweights or elastic elements.

However, the active counterbalance approaches require energy supply to operate, making them unsuitable for devices that need to function during power outages. Additionally, they rely on high-precision sensors, servo controllers, motors, or pumps to achieve satisfactory counterbalance performance. In the passive counterbalance approaches, counterweights are typically positioned in the reverse extension direction of the rotating arm, resulting in low design flexibility. This requires high-density precious metals and significant space for arrangement, and also increases the overall inertia of the rotating arm, leading to operational inconvenience. For the elasticity-based counterbalance approach using elastic elements, it involves employing a spring to pull the movable portion of a revolute joint. This allows the elastic potential energy of the spring and the force potential energy of the movable portion of the revolute joint to interconvert, thereby achieving counterbalance. However, this demands high precision in assembly and matching, making it challenging to achieve sufficiently accurate counterbalance. Further, the passive approaches cannot automatically adapt to changes in gravity or the center of gravity.

To address at least one of the challenges, the embodiments of the present disclosure provide a counterbalance device for a revolute joint and a system including the counterbalance device. The system at least includes a frame and a rotating arm which is supported by the frame and rotatable relative to the frame. To simplify the model, the following description assumes that the force acting on the rotating arm and its connecting members is abstracted as an eccentric force applied to the rotating arm at a location that is offset from the pivot axis of the rotating arm.

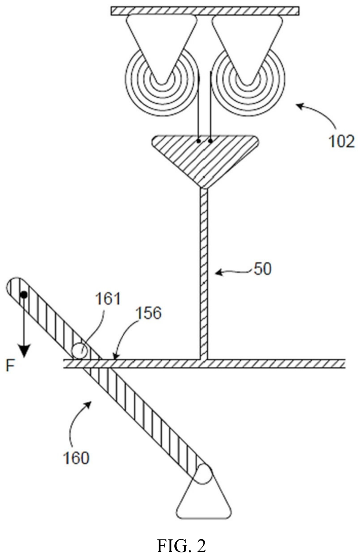

With reference to FIG. 2 to FIG. 4, in some embodiments, the counterbalance device is configured to counterbalance the eccentric force on a rotating arm 160. The rotating arm 160 is supported by a frame (not shown) and rotatable relative to the frame about a first axis. Although the rotating arm 160 depicted in the drawings appears as a straight rod, the specific shape of the rotating arm 160 is not limited herein.

The counterbalance device includes a force output mechanism 102 and a pulling mechanism 50. The force output mechanism 102 is connected to the frame and configured to provide an output force. In some embodiments, the output force is a constant output force. In the embodiments, the first axis is fixed relative to the portion of the force output mechanism 102 connected to the frame (in the following embodiments involving composite joints, the first axis is movable relative to the portion of the force output mechanism 102 connected to the frame). The force output mechanism 102 includes an output end connected to the pulling mechanism 50, so as to apply the output force to the pulling mechanism 50. The pulling mechanism 50 is connected to the frame (e.g., via a guide rail) and translatable relative to the frame along a first line, which is perpendicular to the first axis. The connection between the pulling mechanism 50 and the frame allows the frame to constrain the movement of the pulling mechanism 50 in the direction perpendicular to the first line. In other words, the pulling mechanism 50 has zero displacement or zero displacement component relative to the frame in the direction perpendicular to the first line. The pulling mechanism 50 is further connected to the rotating arm 160, and the pulling mechanism 50 is translatable along the first line with the rotation of the rotating arm 160 about the first axis.

According to the counterbalance device of the present disclosure, the movable pulling mechanism is utilized to connect the force output mechanism and the rotating arm. This allows the force output mechanism with approximately constant output to precisely counterbalance the eccentric force on the rotating arm, without the need to consider the influence of the motion angle of the swim arm on the counterbalance effect. Further, the counterbalance device does not constrain the operational range of the rotating arm and supports its continuous rotation over a full circle.

Specific implementations of the force output mechanism 102 and the pulling mechanism 50 will be described in detail below.

In the embodiments provided herein, to simplify the force model, the first axis is set perpendicular to the direction of the force, and correspondingly, the first line is configured parallel to the direction of the force. It should be understood that the present disclosure is also applicable to other scenarios in which the first axis is transverse to the direction of the force.

### Counterbalance device for Composite Joint

In certain scenarios, a rotating arm may be supported by the frame via composite joints including at least one revolute joint and one prismatic joint, as illustrated in FIG. 1. The movement direction of the prismatic joint is perpendicular to the axis of the revolute joint. For such composite joints, in addition to counterbalance the eccentric force applied to the rotating arm, it is also necessary to counterbalance the force acting on the movable portion of the prismatic joint, thereby making the operation of the prismatic joint smoother and more effortless. Under conditions of complete counterbalance, the movable portion may even be brought into a state of neutral equilibrium. In some cases, the direction of the eccentric force on the rotating arm is the same as that of the force acting on the movable portion of the prismatic joint. For example, when the axis of the revolute joint is transverse to the direction of gravity, the eccentric force on the rotating arm includes the gravity of the rotating arm and its connected structures, and the force acting on the movable portion of the prismatic joint includes the gravity of that movable portion and its connected structures.

Currently, the conventional solution for counterbalance in such composite joints typically involves designing separate counterbalance devices for each joint. That is, an independent counterbalance device is provided for the prismatic joint to counterbalance the force acting on the movable portion, and another independent counterbalance device is provided for the revolute joint to counterbalance the eccentric force on the rotating arm. However, the inventors noted that this solution of using individual counterbalance devices for each joint has the following drawbacks. On one hand, each counterbalance device occupies a certain amount of space inside the robotic arm, which hinders the miniaturization of the composite joints and interferes with the layout design of members and cables within the composite joints. On the other hand, the robotic arm incorporating such composite joints is typically mounted on a suspension platform attached to a base. The two compensation devices significantly increase the overall weight of the robotic arm, thereby imposing a greater load on the suspension platform. This requires higher design standards for the strength and stiffness of the suspension platform, and also leads to an increase in the weight of the equipment chassis.

To address at least one of the aforementioned challenges, the embodiments of the present disclosure provide a counterbalance device for composite joints, and a system including the counterbalance device. The composite joints include at least a prismatic joint and a revolute joint. The system further includes a frame, a first movable member (i.e., the movable portion of the prismatic joint) supported by the frame and translatable relative to the frame, and a second movable member (i.e., the rotating arm) connected to the first movable member and rotatable relative to the first movable member. To simplify the model, the force acting on the rotating arm and its connected members is abstracted as an eccentric force applied on the rotating arm at a location that is offset from the pivot axis of the second movable member.

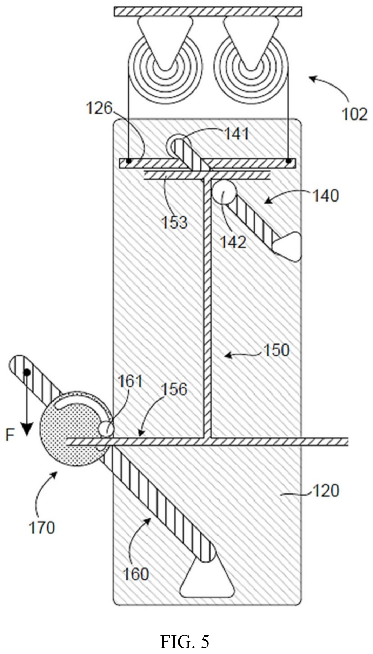

With reference to FIG. 5 to FIG. 7, in some embodiments, the counterbalance device can simultaneously counterbalance the force acting on the first movable member 120 and the eccentric force on the second movable member 160. The first movable member 120 is supported by the frame (not shown) and translatable relative to the frame along the first line, that is, a prismatic joint is formed between the first movable member 120 and the frame, or the first movable member 120 and the frame are connected via a prismatic joint. The second movable member 160 is connected to the first movable member 120 and rotatable relative to the first movable member 120 about the first axis, that is, a revolute joint is formed between the second movable member 160 and the first movable member 120, or the second movable member 160 and the first movable member 120 are connected via a revolute joint. Due to the similar structure and movement of the second movable member 160 to the rotating arm 160 mentioned above, both are designated with the same reference numeral. The first line is perpendicular to the first axis, and the first axis is transverse to the direction of the force. Particularly, the first axis is perpendicular to the direction of the force.

The counterbalance device includes a force output mechanism 102, a distribution mechanism 140/240/340, and a pulling mechanism 150/250/350. The force output mechanism 102 is connected to the frame and configured to provide an output force. In some embodiments, the output force is a constant output force. In the embodiments, the arrangement of the prismatic joint allows the first axis to be translatable relative to the portion of the force output mechanism 102 connected to the frame. The distribution mechanism 140/240/340 is connected to the force output mechanism 102, such that the force output mechanism 102 may apply its output force to the distribution mechanism 140/240/340 at a first load-bearing position of the distribution mechanism 140/240/340. The pulling mechanism 150/250/350 is connected to the distribution mechanism 140/240/340, such that the pulling mechanism 150/250/350 may act on the distribution mechanism 140/240/340 at a second load-bearing position of the distribution mechanism 140/240/340. As such, the distribution mechanism 140/240/340 may distribute the output force provided by the force output mechanism 102 to the first movable member 120 and the second movable member 160, respectively.

The pulling mechanism 150/250/350 is connected to the first movable member 120 (for example, via a guide rail) and translatable relative to the first movable member 120 along the first line. The connection between the pulling mechanism 150/250/350 and the first movable member 120 allows the first movable member 120 to constrain the movement of the pulling mechanism 150/250/350 in the direction perpendicular to the first line. In other words, the pulling mechanism 150/250/350 exhibits zero displacement or zero displacement component relative to the first movable member 120 in the direction perpendicular to the first line. The pulling mechanism 150/250/350 is further connected to the second movable member 160, and the pulling mechanism 150/250/350 is translatable along the first line with the rotation of the second movable member 160 about the first axis.

According to the counterbalance device of the present disclosure, a single force output mechanism 102 is utilized to simultaneously counterbalance the forces acting on both the translatable member and the rotatable member. This simplifies system design, thereby contributing to reduced system weight and minimized system volume.

Specific implementations of the force output mechanism 102, the distribution mechanism 140/240/340, and the pulling mechanism 150/250/350 will be described in detail below.

In the embodiments provided herein, to simplify the force model, the first axis is set perpendicular to the direction of the force, and correspondingly, the first line is configured parallel to the direction of the force. It should be understood that the present disclosure is also applicable to other scenarios in which the first axis is transverse to the direction of the force.

### Force Output Mechanism

The force output mechanism 102 includes an output end connected to the pulling mechanism or the distribution mechanism, such that the force output mechanism 102 may apply its output force to the pulling mechanism or the distribution mechanism. The output end of the force output mechanism 102 is translatable along the first line with the motion of the pulling mechanism or the distribution mechanism, while maintaining the constant output force throughout its movement.

In the present disclosure, the "constant output force" refers to an output force value of the force output mechanism 102 whose fluctuation does not exceed a certain threshold, below which the impact of the fluctuation on the counterbalance device can be ignored. In some embodiments, the threshold may be 0.5%, 1%, 1.5%, or 2% of a theoretical output force value. In some other embodiments, the absolute value of the difference between any two of the maximum output force, the minimum output force, and the average output force of the force output mechanism 102, when taken as a ratio to any one of the maximum output force, the minimum output force, and the average output force of the force output mechanism 102, is less than or equal to 0.5%, 1%, 1.5%, or 2%.

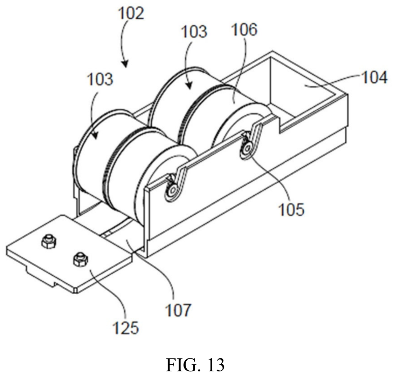

As an implementation, the force output mechanism 102 may include an elastic component configured to provide a constant elastic force, such as a constant-force spring and/or an air spring. The elastic component has an end connected to the frame and the other end connected to the pulling mechanism or the distribution mechanism. As an example of this implementation and referring to FIG. 13, the force output mechanism 102 includes at least one constant-force spring 103. The constant-force spring 103 has its free end connected either directly or indirectly to the pulling mechanism or the distribution mechanism. Detailed structural description will be described in the embodiments of robotic arm.

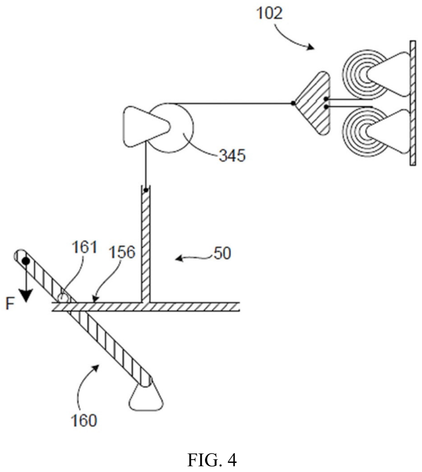

In some embodiments, to enable more flexible arrangement of the force output mechanism 102 within the device, with reference to FIG. 4, the force output mechanism 102 may further include a flexible transmission member (e.g., a cable) and at least one fixed pulley 345. The other end of the elastic component is connected to the pulling mechanism via the flexible transmission member, and the flexible transmission member rides on the at least one fixed pulley 345, thereby enabling the redirection of the tensile force from the force output mechanism 102 to the pulling mechanism. The number of fixed pulleys may be determined based on design requirements. Alternatively, a movable pulley may be provided based on the demand of the output value of the force output mechanism 102.

As another implementation, the force output mechanism 102 may be in the form of a counterweight. For example, the force output mechanism 102 may include a counterweight block, a flexible transmission member, and at least one fixed pulley. The fixed pulley is connected to the frame, the counterweight block is connected to the pulling mechanism via the flexible transmission member, and the flexible transmission member rides on the at least one fixed pulley. The number of fixed pulleys may be determined based on design requirements. Alternatively, a movable pulley may be provided based on the demand of the output value of the force output mechanism 102.

### Pulling mechanism

The pulling mechanism is configured to act on the rotating arm or the second movable member 160 at a first position of the rotating arm or the second movable member 160. The first position has the same displacement as the pulling mechanism along the first line, i.e., the first position is translatable synchronously with the pulling mechanism along the first line. Moreover, due to the change in the angle of the rotating arm or the second movable member 160, the position of the first position relative to the pulling mechanism changes. In other words, the first position is translatable relative to the pulling mechanism along a second line. The second line is transverse to the first line and perpendicular to the first axis. Particularly, the second line is perpendicular to the first line.

It should be noted that, as used herein, a member "acting on" another member refers to that the two members are in direct or indirect contact or connection, such that when a force is applied to one member, it exerts a force on the other, and vice versa. As an implementation, the pulling mechanism may act on the rotating arm or the second movable member 160 via a connecting component 161 mounted on the rotating arm or the second movable member 160, and the position at which the connecting component 161 acts on the rotating arm or the second movable member 160 is the first position. The connecting component 161 may be configured as an integral part of the rotating arm or the second movable member 160, or a discrete member independent of both the rotating arm/second movable member 160 and the pulling mechanism, or a part of a moment arm adjustment mechanism described below. The connecting component 161 is translatable relative to the pulling mechanism along the second line.

The pulling mechanism may be provided with a second load-bearing surface 156 extending along the second line. The connecting component 161 engages the second load-bearing surface 156 and is translatable on the second load-bearing surface 156 along the second line. For example, the connecting component 161 may abut against the second load-bearing surface 156 and is translatable on the second load-bearing surface 156. To reduce friction, the connecting component 161 may slide or roll on the second load-bearing surface 156.

In the illustrated embodiments of the present disclosure, the first position is located above the second load-bearing surface 156. In other embodiments, depending on design requirements, such as to accommodate spatial arrangement of members around the rotating arm or the second movable member 160, the first position may be located below the load-bearing surface 156, where the connecting component 161 has a length extending along the first line. The connecting component 161 has two opposite ends along the first line, with one end acting on the rotating arm or the second movable member 160 at the first position, and the other end engaging the second load-bearing surface 156. The distance between the two opposite ends is design-dependent.

As an example of the connecting component 161, the connecting component 161 may feature an arc-shaped surface circumferentially extending about an axis parallel to the first axis. The arc-shaped surface abuts against the second load-bearing surface 156, such that the arc-shaped surface maintains continuous contact with the second load-bearing surface 156 and moves on the second load-bearing surface 156 during the pivot of the rotating arm or the second movable member 160.

To reduce sliding friction between the connecting component 161 and the second load-bearing surface 156, the arc-shaped surface of the connecting component 161 and the second load-bearing surface 156 may be configured sufficiently smooth, or a lubricant may be applied between the arc-shaped surface and the second load-bearing surface 156. Alternatively, the connecting component 161 may be mounted to the rotating arm or the second movable member 160 and rotatable relative to the rotating arm or the second movable member 160 about a second axis, which passes through the connecting component 161 and is parallel to the first axis, allowing the connecting component 161 to roll on the second load-bearing surface 156. For example, the connecting component 161 may include a roller or a spherical roller.

As another example of the connecting component 161, the connecting component 161 may be configured as a slider having a surface in surface contact with the second load-bearing surface 156. In this configuration, the slider is mounted to the rotating arm or the second movable member 160 and rotatable relative to the rotating arm or the second movable member 160 about a second axis, allowing its surface to maintain continuous surface contact with the second load-bearing surface 156 and move on the second load-bearing surface 156 during the rotation of the rotating arm or the second movable member 160. The second axis passes through the connecting component 161 and is parallel to the first axis.

To reduce sliding friction between the connecting component 161 and the second load-bearing surface 156, the surface of the slider and the second load-bearing surface 156 may be configured sufficiently smooth, or a lubricant may be applied between the surface of the slider and the second load-bearing surface 156.

As yet another example of the connecting component 161, the connecting component 161 may include a slider in any shape and at least two rollers that are connected to the slider and rotatable relative to the slider. The slider is mounted to the rotating arm or the second movable member 160 and rotatable relative to the rotating arm or the second movable member 160 about a second axis that passes through the slider and is parallel to the first axis. The rotation axes of the rollers relative to the slider are parallel to the second axis. The rollers contact the second load-bearing surface 156 and roll on the second load-bearing surface 156.

As an example of the second load-bearing surface 156, the pulling mechanism has a surface configured as the second load-bearing surface 156 extending along the second line. For example, the pulling mechanism is provided with a protruding portion whose upper surface defines the second load-bearing surface 156.

As another example of the second load-bearing surface 156, the pulling mechanism defines a guide slot, and an inner wall of the guide slot includes the second load-bearing surface 156. The connecting component 161 is arranged within the guide slot and movable along the second load-bearing surface 156. For example, the side wall of the guide slot may define the second load-bearing surface 156, namely, the guide slot may have an opening direction oriented perpendicular to the motion plane of the rotating arm or the second movable member 160. For another example, the bottom wall of the guide slot may define the second load-bearing surface 156, namely, the guide slot may have an opening direction oriented parallel to the motion plane of the rotating arm or the second movable member 160. The guide slot helps to prevent the pulling mechanism from separating from the second load-bearing surface 156.

As yet another example of the second load-bearing surface 156, the pulling mechanism is provided with a guide rail (hereinafter referred to as a "fourth guide rail" to distinguish it from other guide rails that may be mentioned subsequently) extending along the second line. The outer wall of the fourth guide rail includes the second load-bearing surface 156, and the connecting component 161 is connected to the fourth guide rail and translatable relative to the fourth guide rail. In the configuration that the connecting component 161 includes a slider, the connecting component 161 engages with the guide rail including the second load-bearing surface 156. The guide rail helps to prevent the pulling mechanism from separating from the second load-bearing surface 156.

### Distribution Mechanism

The distribution mechanism is mounted to the first movable member 120 and rotatable relative to the first movable member 120 about a third axis perpendicular to the first line. The distribution mechanism has a first load-bearing position and a second load-bearing position. The first load-bearing position is the position where the force output mechanism 102 acts on the distribution mechanism, and the second load-bearing position is the position where the pulling mechanism acts on the distribution mechanism. Both the first load-bearing position and the second load-bearing position are arranged external to the third axis, such that the moment generated by the force applied by the force output mechanism 102 at the first load-bearing position about the third axis counterbalances the moment generated by the force applied by the pulling mechanism at the second load-bearing position about the third axis. In some embodiments, the force output mechanism 102 may act on the distribution mechanism at the first load-bearing position via a first connecting member, and the pulling mechanism may act on the distribution mechanism at the second load-bearing position via a second connecting member.

In some embodiments, within a plane perpendicular to the third axis, the projection of the first load-bearing position, the projection of the second load-bearing position, and the projection of the third axis lie on a same line. This maximizes force distribution efficiency in certain scenarios. However, it is not essential. In other embodiments, for example, efficiency may be partially sacrificed to accommodate arrangements of other components.

In some embodiments, the projections of the first load-bearing position and the second load-bearing position lie on the same side of the projection of the third axis on the line. That is, the connecting line between the projection of the first load-bearing position and the projection of the third axis forms a first connecting line, the connecting line between the projection of the second load-bearing position and the projection of the third axis forms a first connecting line, and the angle between the first connecting line and the second connecting line is zero degrees.

In other embodiments, the projection of the third axis lies between the projections of the first load-bearing position and the second load-bearing position on the line. That is, the connecting line between the projections of the first load-bearing position and the third axis forms a first connecting line, the connecting line between the projections of the second load-bearing position and the third axis forms a second connecting line, and the angle between the first connecting line and the second connecting line is 180 degrees. In the embodiments, it needs to employ a direction-changing mechanism between the first load-bearing position and the force output mechanism 102, or between the second load-bearing position and the pulling mechanism, to alter the direction of force applied at the first load-bearing position or the second load-bearing position, thereby achieving moment equilibrium.

In some embodiments, the distance between the first load-bearing position and the third axis is adjustable, and/or the distance between the second load-bearing position and the third axis is adjustable, thereby regulating the proportional relationship between the two moments to accommodate different design requirements and satisfy scenarios with varying compensation forces. The method of adjustment depends on the specific implementation of the distribution mechanism and will be described in detail below.

The distribution mechanism may be implemented in various forms. Several implementation examples are listed here for illustration.

Referring to FIG. 5, as a first implementation, the distribution mechanism 140 has an end connected to the first movable member 120 and rotatable relative to the first movable member 120, such that the distribution mechanism 140 is rotatable relative to the first movable member 120 about the third axis. The distribution mechanism 140 may be configured in the form of a lever for torque distribution. As shown in FIG. 5, the distribution mechanism 140 is configured as an elongated straight rod. However, in other embodiments, the distribution mechanism 140 may be configured as a curved, bent, or other shaped rod to achieve spatial avoidance as needed. The distribution mechanism 140 is not necessarily limited to the rod-shaped configuration, and any other shape capable of performing the function of the lever may be employed.

The first connecting member includes a first load-bearing member 126 and a first load-applying member 141. The first load-bearing member 126 is connected to the output end of the force output mechanism 102, and the first load-applying member 141 is connected to the first load-bearing position of the distribution mechanism 140. The first load-bearing member 126 is provided with a first load-bearing surface 127. The first load-applying member 141 acts on the first load-bearing surface 127 and is translatable relative to the first load-bearing surface 127 along a fourth line, which is transverse to the first line. Specifically, the fourth line is perpendicular to the first line.

The second connecting member includes a second load-bearing member 142 and a second load-applying member 153. The second load-bearing member 142 is connected to the second load-bearing position of the distribution mechanism 140. The second load-applying member 153 is connected to the pulling mechanism 150. The second load-applying member 153 is provided with a load-applying surface 154. The load-applying surface 154 acts on the second load-bearing member 142, and the second load-bearing member 142 is translatable relative to the load-applying surface 154 along a fifth line, which is transverse to the first line. Specifically, the fifth line is perpendicular to the first line.

The first load-applying member 141 and/or the second load-bearing member 142 are detachably mounted on the distribution mechanism 140, such that the installation positions of the first load-applying member 141 and/or the second load-bearing member 142 on the distribution mechanism 140 may be adjusted according to actual needs. Consequently, the distance between the first load-bearing position and the third axis, and/or the distance between the second load-bearing position and the third axis, are adjustable. In some embodiments, the distribution mechanism 140 may be provided with multiple mounting positions to facilitate position adjustment of the first load-applying member 141 and/or the second load-bearing member 142. In other embodiments, the positions of the first load-applying member 141 and/or the second load-bearing member 142 may be adjusted via a moment arm adjustment mechanism that will be described below.

The relationship between the first load-applying member 141 and the first load-bearing surface 127 in structure and function may be configured to be similar to that between the connecting component 161 and the second load-bearing surface 156 of the pulling mechanism 150. In some embodiments, the first load-applying member 141 may include at least one first roller 141 and/or a first slider. In some embodiments, the first load-bearing surface 127 may be the upper surface of the first load-bearing member 126, or an inner wall surface of a guide slot defined in the first load-bearing member 126, or an outer wall surface of a guide rail provided on the first load-bearing member 126.

The relationship between the second load-bearing member 142 and the load-applying surface 154 in structure and function may be configured to be similar to that between the connecting component 161 and the second load-bearing surface 156 of the pulling mechanism 150, but with opposite installation positions. In some embodiments, the second load-bearing member 142 may include at least one second roller 142 and/or a second slider. In some embodiments, the load-applying surface 154 may be the lower surface of the second load-applying member 153, or an inner wall surface of a guide slot defined in the second load-applying member 153, or an outer wall surface of a guide rail provided on the second load-applying member 153.

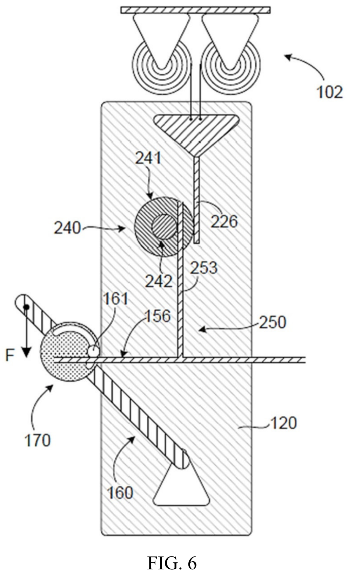

Referring to FIG. 6, as a second implementation, the distribution mechanism 240 is provided with a first peripheral member 241 and a second peripheral member 242 that both extend circumferentially about the third axis. The first peripheral member 241 and the second peripheral member 242 are offset along the third axis. The first load-bearing position is located on the first peripheral member 241, and the second load-bearing position is located on the second peripheral member 242.

Both the first peripheral member 241 and the second peripheral member 242 include teeth which are circumferentially arranged. The first connecting member 226 includes teeth arranged along the first line and meshes with the first peripheral member 241. The second connecting member 253 includes teeth arranged along the first line and meshes with the second peripheral member 242. In some embodiments, the distribution mechanism 240 may include two gears, with each detachably fixed to a rotating shaft. The distance between the first load-bearing position and the third axis, and/or the distance between the second load-bearing position and the third axis, may be adjusted by way of replacing gears with different outer diameters, thereby enabling moment distribution adjustment. In other embodiments, the distribution mechanism 240 may include two gears which are connected via a transmission (e.g., a mechanical continuously variable transmission) to adjust the relative ratio of the working radii of the two gears.

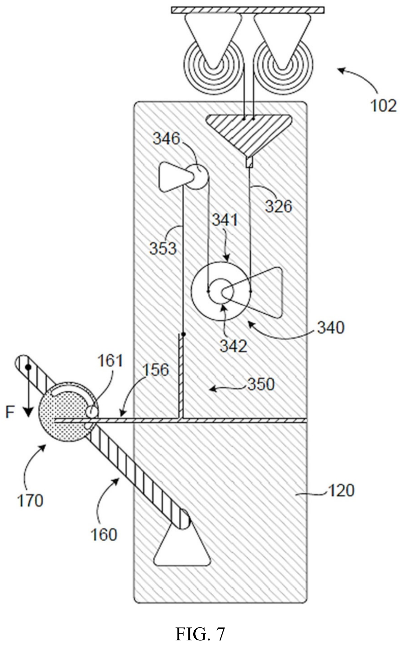

Referring to FIG. 7, as a third implementation and a variant of the second implementation, the distribution mechanism 340 includes a first peripheral member 341 and a second peripheral member 342 both extending circumferentially about the third axis. The first peripheral member 341 and the second peripheral member 342 are offset along the third axis. The first load-bearing position is located on the first peripheral member 341, and the second load-bearing position is located on the second peripheral member 342.

Different from the second implementation, the third implementation employs a flexible transmission approach. In some embodiments, the first peripheral member 341 defines a first cable groove 341 extending circumferentially. The first connecting member 326 includes a first cable 326 which rides on the distribution mechanism 340 along the first cable groove 341. The second peripheral member 342 defines a second cable groove 342 extending circumferentially. The second connecting member 353 includes a second cable 353 which rides on the distribution mechanism 340 along the second cable groove 342. In some embodiments, the distribution mechanism 340 may include two pulleys, with each detachably fixed to a rotating shaft. The distance between the first load-bearing position and the third axis, and/or the distance between the second load-bearing position and the third axis, may be adjusted by way of replacing pulleys with different outer diameters, thereby enabling moment distribution adjustment. In other embodiments, the distribution mechanism 340 may include two pulleys which are connected via a transmission (e.g., a mechanical continuously variable transmission) to adjust the relative ratio of the working radii of the two pulleys.

The flexible transmission approach may alternatively be implemented via, for example, a synchronous pulley mechanism, a steel band mechanism, or a sprocket-chain mechanism. Thus, the cables and grooves in the third implementation may be substituted to get a new implementation.

The foregoing introduces basic concepts of several implementations of the distribution mechanism. More detailed descriptions of structure of the distribution mechanism will be provided below in conjunction with the robotic arm embodiments.

It should be understood that the distribution mechanism in the present disclosure is not limited to the three implementations described above. Other structures capable of achieving moment distribution are also included within the implementations of the distribution mechanism.

### Moment Arm Adjustment Mechanism

In certain situations, the magnitude and/or the application point of the force acting on the entire assembly of the rotating arm/second movable member 160 and its connected members may change. For example, in the case where the force is gravity, when the weight of other members connected to the rotating arm or the second movable member 160 changes, or when the overall center of gravity shifts due to the movement of other members relative to the rotating arm or the second movable member 160, this results in a change in the gravitational moment relative to the first axis.

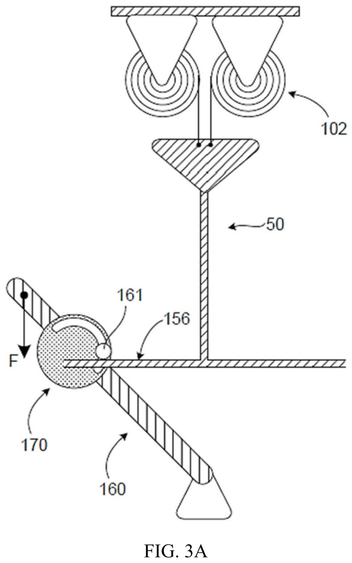

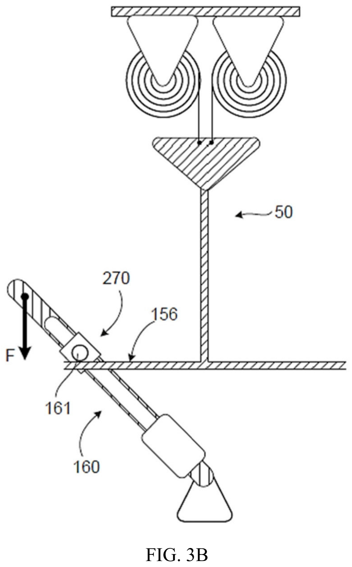

To allow the counterbalance device of the present disclosure to automatically adapt to such changes, the counterbalance device according to the embodiments may further include a moment arm adjustment mechanism 170. The moment arm adjustment mechanism 170 is configured to change the relative position between the connecting component 161 and the rotating arm or the second movable member 160 to adjust the distance of the connecting component 161 relative to the first axis. Embodiments of the moment arm adjustment mechanism are illustrated in FIG. 3A and FIG. 3B.

The moment arm adjustment mechanism 170 includes a driving member 171. The driving member 171 is configured to directly or indirectly move the connecting component 161 relative to the rotating arm or the second movable member 160, to change the distance between the connecting component 161 and the first axis. In some embodiments, the driving member 171 may include a rotary motor or a linear motor. In some embodiments, the connecting component 161 is movable in a path lying on a plane perpendicular to the first axis.

The path of movement of the connecting component 161 may be curved. In the embodiments as shown in FIG. 3A, the projection, on the plane on which the arc lies, of the connecting line between the application point of the eccentric force and the first axis passes through the center of the arc. Alternatively, the path of movement of the connecting component 161 may be straight. In the embodiments as shown in FIG. 3B, the path coincides with the projection, on the plane perpendicular to the first axis, of the connecting line between the application point of the eccentric force and the first axis. Alternatively, the path of movement of the connecting component 161 may be any other curved line or polygonal line.

In some embodiments, the driving member 171 may drive the connecting component 161 via a transmission member 173. That is, the transmission member 173 connects the driving member 171 and the connecting component 161. In some embodiments, the transmission member 173 may include at least one of gear transmission, worm gear transmission, pulley transmission, and screw transmission.

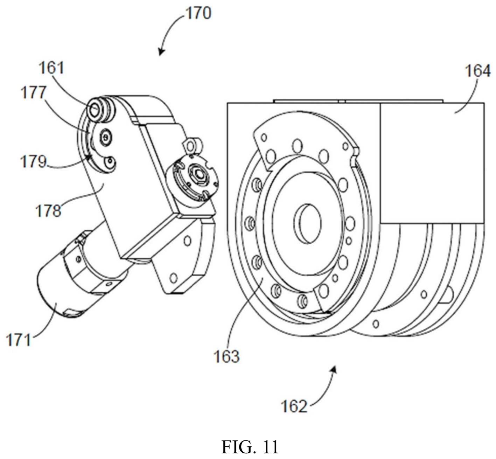

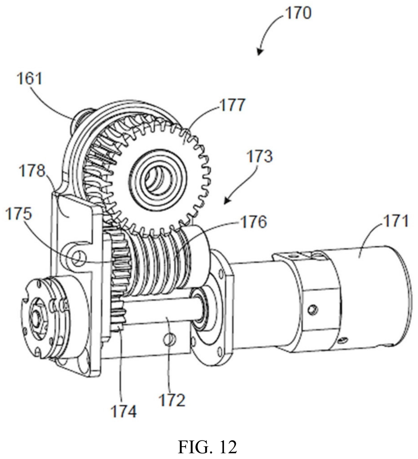

The embodiment shown in FIG. 3A may refer to FIG. 11 and FIG. 12, which illustrates a detailed implementation. The driving member 171 is configured as a rotary motor including an output shaft 172. The transmission member 173 includes a first gear portion 174, a second gear portion 175, a worm portion 176, and a worm gear portion 177. The first gear portion 174 is fitted on the output shaft 172. The second gear portion 175 is meshed with the first gear portion 174. The second gear portion 175 and the worm portion 176 rotate coaxially. The worm gear portion 177 is meshed with the worm portion 176 and is rotatable about a fourth axis. The output shaft 172 of the rotary motor is rotatable about an axis perpendicular to the fourth axis. The connecting component 161 is disposed on the worm gear portion 177 and offset from the axis of the worm gear portion 177. Consequently, the driving member 171 moves the connecting component 161 along an arc path around the fourth axis. As a variant, the gear transmission between the first gear portion 174 and second gear portion 175 may be substituted to belt-pulley transmission. As another variant, the worm gear portion 177 may be directly fitted on the output shaft 172, thereby omitting the first gear portion 174 and the second gear portion 175. As yet another variant, a gear may be provided between the first gear portion 174 and the second gear portion 175.

For the embodiment shown in FIG. 3B, as an implementation, the driving member 171 includes a rotary motor, and the transmission member 173 includes a screw and a nut threadedly engaged with the screw. The screw is connected to the output shaft 172 of the rotary motor, and the connecting component 161 is mounted to the nut and rotatable relative to the nut. Consequently, the driving member 171 moves the connecting component 161 along the screw. As another implementation, the driving member 171 includes a linear motor, and the connecting component 161 is connected to the mover of the linear motor, omitting the lead screw and nut.

The moment arm adjustment mechanism may also be applied to the distribution mechanism. For example, as the first implementation of the distribution mechanism, the moment arm adjustment mechanism may be provided between the first load-applying member 141 and the distribution mechanism 140, and/or between the second load-bearing member 142 and the distribution mechanism 140.

### Robotic Arm with Counterbalance device

The counterbalance device described above may be applied to various scenarios requiring counterbalance. The following description uses a robotic arm as an example to provide an exemplary description of the structural details and application of the counterbalance device. It should be understood that the following description does not limit the scope of protection of the present disclosure.

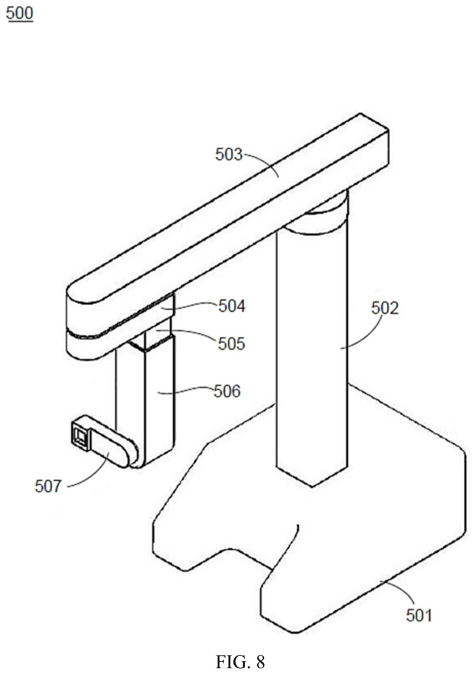

As shown in FIG. 8, the counterbalance device is applied to a slave operating apparatus 500 in a medical system, such as a patient-side robotic arm system for surgical robots. The medical system may further include a master operating apparatus (console) and an imaging-display apparatus, neither of which is shown in the drawing. The slave operating apparatus 500 includes a base 501, a column 502, a transverse link, a longitudinal link, and a movable link 507.

The column 502 has a bottom mounted on the base 501 and a top connected to the transverse link. In the embodiments as shown in FIG. 8, the transverse link and the column 502 are connected via a revolute joint and a prismatic joint, to allow the transverse link to rotate and move up and down relative to the base 501. In other embodiments (not shown in the drawings), the column 502 itself may be provided with a revolute joint and/or a prismatic joint; or, the column 502 and the base 501 may be connected via a revolute joint and/or a prismatic joint, as long as the transverse link is allowed to rotate and move up and down relative to the base 501. In the embodiments as shown in FIG. 8, the transverse link includes two links 503, 504 that are connected via a revolute joint, such that the link 504 is rotatable relative to the link 503. In other embodiments (not shown in the drawings), the transverse link may include one link, or may include three or more links with adjacent links connected via a revolute joint or a prismatic joint.

The transverse link includes an end link connected to the longitudinal link. The longitudinal link includes a portion 506 movable relative to the end link of the transverse link (hereinafter referred to as a movable portion), and a portion 505 fixed relative to the end link of the transverse link (hereinafter referred to as a fixed member). The movable portion 506 is connected to the fixed portion 505 via a prismatic joint, such that the movable portion 506 is translatable vertically relative to the transverse link. Since the fixed portion 505 is fixed relative to the end link of the transverse link, the fixed portion 505 may be considered as part of the end link of the transverse link. The movable link 507 is connected to the movable portion 506 of the longitudinal link via a revolute joint, such that movable link 507 is pivotable relative to the longitudinal link on a vertical plane. That is, the movable link 507 is connected to the transverse link via a revolute joint and a prismatic joint, such that movable link 507 is translatable vertically relative to the transverse link and pivotable within the vertical plane.

In the scenario shown in FIG. 8, the gravity of the movable portion 506 of the longitudinal link and the gravity of the movable link 507 and its connected structures need to be counterbalanced. Thus, in such scenario, the counterbalance device may be regarded as a gravity compensation device.

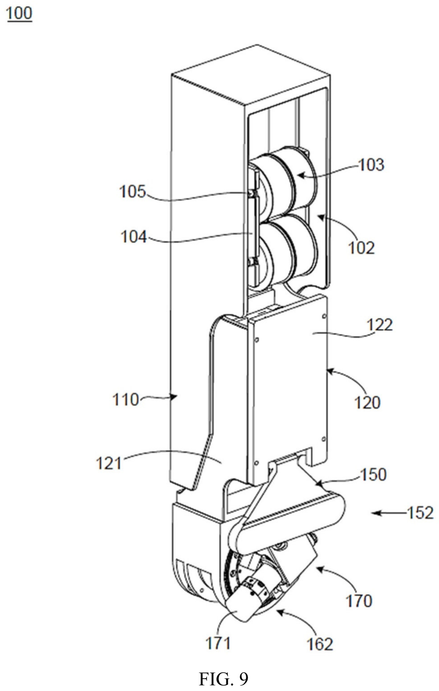

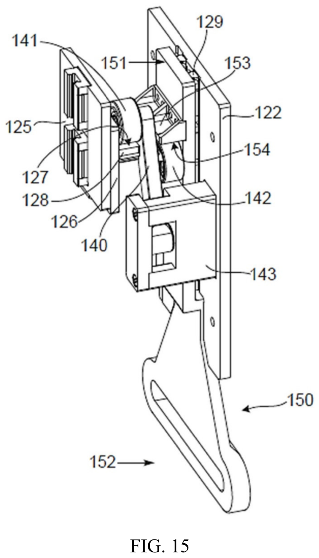

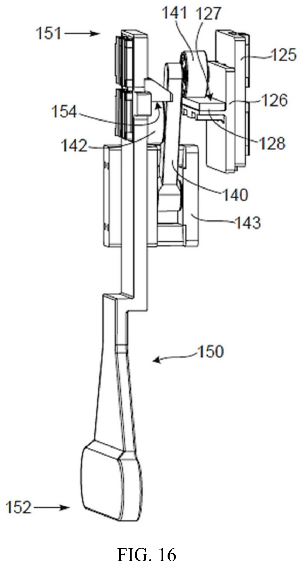

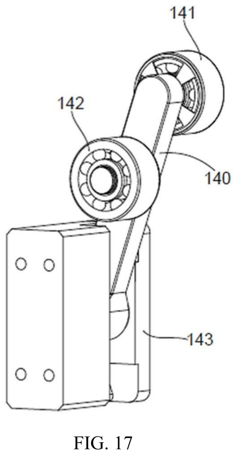

The detailed structure of the robotic arm will be described below with reference to FIG. 9 to FIG. 17. The robotic arm according to the embodiments may include at least the movable portion 506 of the longitudinal link of the slave operating apparatus 500, and a revolute joint which connects the movable member 506 and the movable link 507.

Referring to FIG. 9 to FIG. 17, the robotic arm 100 includes a first link 120 and a second link 110. The second link 110 is connected to a frame (which may be understood as a combination of the base 501, the column 502, and the transverse link). In some embodiments, the second link 110 may be fixed to the end link of the transverse link via bolts, welding, or other means. In other embodiments, the second link 110 may not be part of the robotic arm 100, but rather be configured as an integral part of the end link of the transverse link, i.e., the second link 110 may be part of the frame.

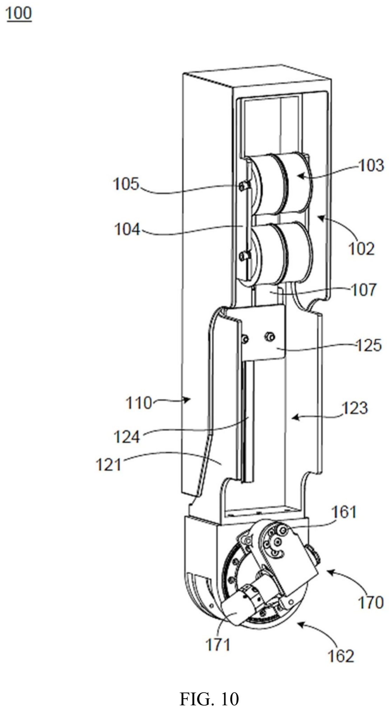

In the embodiments shown in FIG. 9 and FIG. 10, the second link 110 defines a second accommodating cavity, and a third guide rail (not shown) extending along the first line is provided inside the second accommodating cavity. The first link 120 is at least partially accommodated in the second accommodating cavity and connected to the third guide rail, allowing the first link 120 to translate relative to the second link 110 (i.e., relative to the frame) along the first line. In other embodiments, the second link 110 may be at least partially accommodated in a first accommodating cavity defined in the first link 120 and connected to a guide rail provided inside the first accommodating cavity. The first link 120 may correspond to the first movable member 120.

The first link 120 is further connected to a revolute joint 162 which includes a rotating member 163. The rotating member 163 is rotatable relative to the first link 120 about a first axis that is perpendicular to the first line. The rotating member 163 may be connected to other links (e.g., the movable link 507), a joint, or a surgical instrument, etc. The rotating member 163 and its connected members may be regarded as the rotating arm or the second movable member 160 subjected to the eccentric force. In the embodiments, the rotating member 163 may be configured as a flange shaft 163. The flange shaft 163 has a shaft portion connected to the first link 120 and a flange portion facilitating the mounting or connection of other members. The revolute joint 162 further includes a mounting member 164 fixed to the first link 120. The rotating member 163 is mounted on the mounting member 164 and rotatable relative to the mounting member 164. In other embodiments, the rotating member 163 may be directly mounted to the first link 120 and rotatable relative to the first link 120. For example, the rotating member 163 is received within the first accommodating cavity of the first link 120 and rotatable relative to the first link 120.

The robotic arm 100 further includes a counterbalance device. In the embodiments, the counterbalance device may be the aforementioned counterbalance device for composite joints, which counterbalances the gravity of the first link 120 and its connected members, as well as the gravitational torque generated by the rotating member 163 and its connected members. In other embodiments, the counterbalance device may be the aforementioned counterbalance device for a revolute joint, which counterbalances the gravitational torque generated by the rotating member 163 and its connected members. In some embodiments, the counterbalance device is at least partially accommodated within the first accommodating cavity of the first link 120 and the second accommodating cavity of the second link 110.

In the embodiments of the present disclosure, the counterbalance device includes a force output mechanism 102, a distribution mechanism 140/240/340, and a pulling mechanism 150/250/350. The force output mechanism 102 acts on the distribution mechanism 140/240/340 at a first load-bearing position of the distribution mechanism 140/240/340 via a first connecting member, and the pulling mechanism acts on the distribution mechanism 140/240/340 at a second load-bearing position of the distribution mechanism 140/240/340 via a second connecting member. As previously described, the design concepts, implementation forms, technical effects, as well as the interconnection and collaboration between the force output mechanism 102, the distribution mechanism 140/240/340, and the pulling mechanism 150/250/350 have already been described and will not be reiterated herein. The following will provide an exemplary description of the structural details of these mechanisms and their structural relationships with other components of the robotic arm 100, to further illustrate how the counterbalance device counterbalances the gravity within the robotic arm 100.

Referring to FIG. 9, FIG. 10, and FIG. 13, the force output mechanism 102 may include a mounting base 104 and at least one constant-force spring 103. The mounting base 104 is fixed relative to the second link 110. In some embodiments, the mounting base 104 is received within the second accommodating cavity of the second link 110. The mounting base 104 features two spaced-apart side walls and a third accommodating cavity defined between the two side walls. The constant-force spring 103 is at least partially received within the third accommodating cavity. The constant-force spring 103 includes a drum 106 and a spring band 107 wound on the drum 106. Each of the two side walls of the mounting base 104 defines an inclined notch. A mounting shaft 105 is arranged across the inclined notches in the two side walls, and the drum 106 is fixed over the mounting shaft 105. The constant-force spring 103 has a free end (i.e., the outermost end of the spring band 107) connected either directly or indirectly to the pulling mechanism or the distribution mechanism. In the embodiments, the free end of the constant-force spring 103 is connected to the pulling mechanism or the distribution mechanism via a sliding member 125. The number of the constant-force springs 103 may be selected based on the requirement of adjusting the output force of the force output mechanism 102. In some embodiments, the force output mechanism 102 may include multiple constant-force springs 103 arranged in parallel. Additionally, the constant-force spring 103 may be formed by recoiling multiple layers of the spring band 107, allowing the output force of the force output mechanism 102 to be adjusted by increasing or decreasing the number of layers of the spring band 107.

Referring to FIG. 9 and FIG. 10, the first link 120 may include a main frame portion 121 and a secondary frame portion 122. The main frame portion 121 is connected to the secondary frame portion 122, and a first accommodating cavity 123 is defined between the main frame portion 121 and the secondary frame portion 122. The distribution mechanism 140, the first connecting member, the second connecting member, and the pulling mechanism are at least partially located within the first accommodating cavity 123. The main frame portion 121 has an inner side surface provided with a first guide rail 124 extending along the first line. A sliding member 125 is arranged on the first guide rail 124 and translatable relative to the first guide rail 124. The sliding member 125 is connected to the output end of the force output mechanism 102. In the embodiments, the free end of the constant-force spring 103 is inserted into the first accommodating cavity 123 and connected to the sliding member 125. Thus, the constant-force spring 103 is allowed to be retracted or released as the sliding member 125 translates along the first guide rail 124. The secondary frame portion 122 has an inner side surface provided with a second guide rail 129 extending along the first line. The pulling mechanism is arranged on the second guide rail 129 and translatable relative to the second guide rail 129.

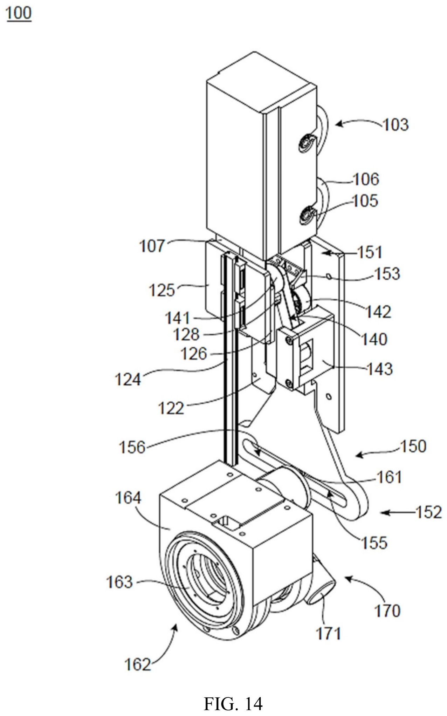

In the first example illustrated in FIG. 14 to FIG. 17, the distribution mechanism 140 may adopt, as shown in FIG. 5, the aforementioned first implementation. The first load-bearing member 126 is mounted to the first link 120 and translatable relative to the first link 120 along the first line. The first load-bearing member 126 has a first load-bearing surface 127. The force output mechanism 102 is connected to the first load-bearing member 126 and is configured to provide a constant output force to the first load-bearing member 126. The distribution mechanism 140 is connected to the first link 120 and rotatable relative to the first link 120 about a third axis which is perpendicular to the first line. The distribution mechanism 140 is provided with a first load-applying member 141 and a second load-bearing member 142. The first load-applying member 141 acts on the first load-bearing surface 127 of the first load-bearing member 126, and is translatable relative to the first load-bearing surface 127 along a fourth line which is transverse to the first line. The second load-applying member 153 is connected to the pulling mechanism 150 to translate with the pulling mechanism 150 along the first line. The second load-applying member 153 has a load-applying surface 154 acting on the second load-bearing member 142. The second load-bearing member 142 is translatable relative to the load-applying surface 154 along a fifth line which is transverse to the first line. Particularly, the fourth line is parallel to the fifth line and perpendicular to the first line.

The distance between the first load-applying member 141 and the third axis is adjustable, and/or the distance between the second load-bearing member 142 and the third axis is adjustable. Therefore, the proportional relationship between the two distances can be adjusted according to the requirements of moment distribution.

In a first example, within a plane perpendicular to the third axis, the connecting line between the connection position of the first load-applying member 141 with the distribution mechanism 140 and the third axis forms a first connecting line, and the connecting line between the connection position of the second load-bearing member 142 with the distribution mechanism 140 and the third axis forms a second connecting line. The included angle between the first connecting line and the second connecting line may be zero degrees. This is beneficial for simplifying the design of the distribution mechanism. In other examples, the included angle between the first connecting line and the second connecting line may be different values.

In the first example, the distribution mechanism 140 is configured as a lever. The lever 140 has one end mounted to the first link 120 and rotatable relative to the first link 120 about a fourth axis. In some embodiments, a connection seat 143 is provided within the first accommodating cavity 123 of the first link 120, and the connection seat 143 is fixed to the inner side surface of the secondary frame portion 122. One end of the distribution mechanism 140 is connected to the connection seat 143 and rotatable relative to the connection seat 143.

The first load-applying member 141 and the second load-bearing member 142 are arranged on the distribution mechanism 140. In some embodiments, the first load-applying member 141 and the second load-bearing member 142 may be spaced apart in the length direction of the lever, and/or arranged on both sides of the lever in the thickness direction of the lever. The thickness direction of the lever is parallel to the fourth axis. The positions of the first load-applying member 141 and the second load-bearing member 142 in the length direction of the lever may be adjusted according to moment distribution requirements. This adjustment may be achieved manually or, for instance, by providing a moment arm adjustment mechanism between the first load-applying member 141 and the lever and/or between the second load-bearing member 142 and the lever. Positioning the first load-applying member 141 and the second load-bearing member 142 on both sides of the lever in the thickness direction of the lever helps prevent undesired interference between the first load-bearing member 126 and the second load-applying member 153 when the length of the lever is reduced, thereby reducing space occupied by the lever.

In the first example, the first load-applying member 141 includes one first roller 141. The first roller 141 is configured to roll on the first load-bearing surface 127, with its rotation axis parallel to the third axis. In other examples, the first load-applying member 141 may include multiple first rollers. In another example, the first load-applying member 141 may include a first slider, which is connected to the distribution mechanism 140 and rotatable relative to the distribution mechanism 140. The first slider is rotatable about an axis parallel to the third axis. The first slider has a surface in contact with the first load-bearing surface 127 and is configured to slide on the first load-bearing surface 127.

In the first example, the second load-bearing member 142 includes one second roller 142. The second roller 142 is configured to roll on the load-applying surface 154, with its rotation axis parallel to the third axis. In other examples, the second load-bearing member 142 may include multiple second rollers. In another example, the second load-bearing member 142 may include a second slider connected to the distribution mechanism 140 and rotatable relative to the distribution mechanism 140. The second slider is rotatable about an axis parallel to the third axis. The second slider has a surface in contact with the load-applying surface 154 and is configured to slide on the load-applying surface 154.

The first load-bearing member 126 is mounted to the first link 120 and translatable relative to the first link 120 along the first line. In some embodiments, the first load-bearing member 126 is fixed to the sliding member 125. As such, the first load-bearing member 126 is connected to the first link 120 via the sliding member 125 and the first guide rail 124, and it is translatable relative to the first link 120. In the first example, the first load-bearing member 126 is provided with a first protruding portion 128 protruding towards the first accommodating cavity 123. The first protruding portion 128 has an upper surface perpendicular to the first line, and the upper surface defines the first load-bearing surface 127.

The second load-applying member 153 is connected to the pulling mechanism 150 so as to translate with the pulling mechanism along the first line. In some embodiments, the second load-applying member 153 is fixed to the pulling mechanism 150 and thereby translating synchronously with the pulling mechanism 150 on a second guide rail 128 along the first line. In the first example, the second load-applying member 153 is provided with a second protruding portion protruding towards the first accommodating cavity 123. The second protruding portion has a lower surface perpendicular to the first line, and the lower surface defines the load-applying surface 154.

Further, the connection seat 143 is offset from the first load-bearing member 126 and the second load-applying member 153 in the direction perpendicular to the first line, such that the connection seat 143 avoids undesired interference with the movement of the first load-bearing member 126 and the second load-applying member 153 within the pivot range of the lever.

In the first example, the pulling mechanism 150 includes a first end 151 and a second end 152 facing each other along the first line. The second load-applying member 153 is arranged at the first end 151. In some embodiments, the second load-applying member 153 is fixed to the first end 151, so as to move synchronously with the first end 151 of the pulling mechanism 150. The second end 152 is connected to the rotating member 163. In some embodiments, at the second end 152 of the pulling mechanism 150, the pulling mechanism 150 acts on the rotating member 163 via at least the connecting component 161. Further, the moment arm adjustment mechanism 170 may be disposed between the connecting component 161 and the rotating member 163, which will be described below. The connecting component 161 is translatable relative to the pulling mechanism 150 along the second line which is transverse to the first line and perpendicular to the first axis. In some embodiments, the second load-bearing surface 156 extending along the second line is provided at the second end 152 of the pulling mechanism 150. The connecting component 161 presses against the second load-bearing surface 156 and is movable on the second load-bearing surface 156. The first end and the second end refer to relative positions, not necessarily absolute extremities, and may denote positions near the ends, or other locations.

In the first example, the second load-applying member 153 protrudes beyond the extending plane of the pulling mechanism 150 and presses against the second load-bearing member 142. Accordingly, the pulling mechanism 150 may be construed as being suspended from the distribution mechanism 140 via the second connecting member (i.e., the second load-applying member 153 and the second load-bearing member 142).

In the first example, the pulling mechanism 150 defines a guide slot 155. The guide slot 155 has an inner wall, which includes the second load-bearing surface 156. The connecting component 161 is arranged within the guide slot 155 and movable relative to the guide slot 155. In some embodiments, the pulling mechanism 150 is configured with a structure that is wider at the bottom and narrower at the top, and the guide slot 155 extends in the width direction, i.e., along the second line, at the second end 152. In other examples, the pulling mechanism 150 is provided with a fourth guide rail extending along the second line. The fourth guide rail has an outer wall, which includes the second load-bearing surface 156. The connecting component 161 is connected to the fourth guide rail and movable relative to the fourth guide rail.

In the first example, the connecting component 161 includes one third roller. The third roller is configured to roll within the guide slot 155 along the second load-bearing surface 156, with its rotation axis parallel to the first axis. In other examples, the connecting component 161 may include multiple third rollers. In another example, the connecting component 161 may include a third slider connected to the distribution mechanism 140 and rotatable relative to the distribution mechanism 140. The third slider is rotatable about an axis parallel to the third axis. The third slider has a surface in contact with the second load-bearing surface 156 and is configured to slide on the second load-bearing surface 156.

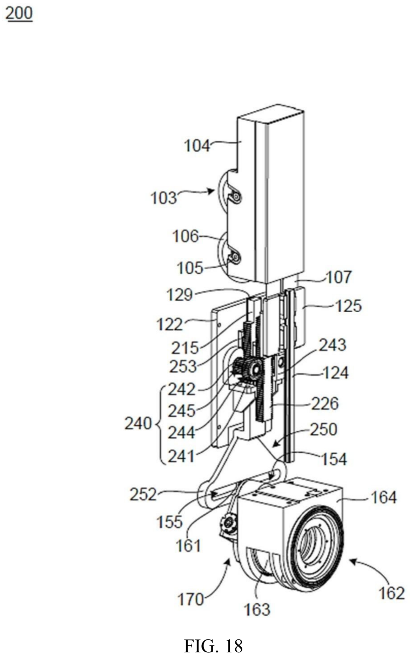

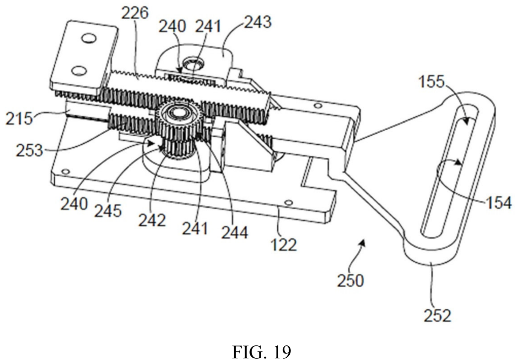

In the second example shown in FIG. 18 and FIG. 19, the distribution mechanism 240 may adopt, as shown in FIG. 6, the second implementation. The distribution mechanism 240 is connected to the first link 120 and rotatable relative to the first link 120 about the fourth axis, and the third axis is perpendicular to the first line. The distribution mechanism 240 includes a first tooth portion 241 and a second tooth portion 242 that are offset along the third axis. Each of the first tooth portion 241 and the second tooth portion 242 includes teeth arranged circumferentially about the third axis. The first connecting member 226 is connected to the first link 120 and translatable relative to the first link 129 along the first line. The first connecting member 226 is further connected to the force output mechanism 102. The first connecting member 226 includes teeth arranged along the first line, and the first connecting member 226 meshes with the first tooth portion 241. The second connecting member 253 is connected to the pulling mechanism 250 so as to translate along the first line when driven by the pulling mechanism 250. The second connecting member 253 includes teeth arranged along the first line, and the second connecting member 253 meshes with the second tooth portion 242.

The distance between the first tooth portion 241 and the third axis may be equal to or different from the distance between the second tooth portion 242 and the third axis. The proportional relationship between the two distances may be adjusted according to the requirements of moment distribution.

In the second example, within the plane perpendicular to the third axis, the connecting line between the connection position of the first connecting member 226 with the first tooth portion 241 and the third axis forms a first connecting line, and the connecting line between the connection position of the second connecting member 253 with the second tooth portion 242 and the third axis forms a second connecting line. The included angle between the first connecting line and the second connecting line is zero degrees. This is advantageous for simplifying the design of the distribution mechanism. In other examples, the included angle between the first connecting line and the second connecting line may be different values.

In the second example, the distribution mechanism 240 includes a first gear 244 and a second gear 245 that rotate synchronously and are coaxially arranged. The first gear 244 and the second gear 245 are mounted to the first movable member 120 and rotatable relative to the first movable member 120 about the third axis. The first gear 244 has the first tooth portion 241, and the second gear 245 has the second tooth portion 242. The diameter of the first gear 244 and the diameter of the second gear 245 may or may not be equal. The ratio between the diameter of the first gear 244 and the diameter of the second gear 245 may be selected according to requirements of moment distribution.

Further, the first gear 244 and/or the second gear 245 is detachably mounted to the first movable member 120, thereby facilitating the replacement of the first gear 244 and/or the second gear 245. For example, gears with different diameters may be used to adapt to different moment distribution requirements. In some embodiments, the first gear 244 and the second gear 245 are connected via a transmission (e.g., a mechanical continuously variable transmission) to adjust the relative ratio of the working radii of the two gears.

In the second example, the distribution mechanism 240 further includes a housing 243. The first tooth portion 241 and the second tooth portion 242 are received within the housing 243. The first tooth portion 241 at least partially protrudes from the housing 243 to mesh with the first connecting member 226, and the second tooth portion 242 at least partially protrudes from the housing 243 to mesh with the second connecting member 253. The housing 243 is fixed to the inner side surface of the secondary frame portion 122.

In the second example, the first connecting member 226 may be directly connected to the sliding member 125. In some embodiments, the first connecting member 226 is fixed to the sliding member 125. As such, the first connecting member 226 is connected to the first link 120 via the sliding member 125 and the first guide rail 124, and it is translatable relative to the first link 120. In some embodiments, the first connecting member 226 may be configured as a rack, which has a length extending parallel to the first line. The rack includes a side surface having teeth arranged in its length direction, and another side surface connected to the sliding member 125 that is perpendicular to the side surface.

In the second example, the pulling mechanism 250 includes a first connection member 215 and a second connection member 252. The first connection member 215 extends at least along the first line and is connected to the second guide rail 128. The second connection member 252 extends at least transversely to the first line and is configured to define the second load-bearing surface 156. The first connection member 215 includes two ends along the first line. The second connection member 252 is arranged at the end of the first connection member 215 close to the rotating member 163. In the second example, the general configuration of the pulling mechanism 250, the arrangement of the second load-bearing surface 156, and the arrangement of the connecting component 161 are similar to those of the pulling mechanism 150 in the first example, with the primary difference lying in the arrangement of the second connecting member 253 on the pulling mechanism 150. In the second example, both sides of the extending plane of the pulling mechanism 250 is provided with the second connecting member 253. In some embodiments, a side edge of the first connection member 215 is configured as the second connecting member 253.

To enhance the stability of the connection between the distribution mechanism 240, the pulling mechanism 250, and the force output mechanism 102, multiple distribution mechanisms 240 may be provided on the first link 120. Accordingly, the quantities of the first connecting members 226 and the second connecting members 253 may be adjusted correspondingly. In the second example, two distribution mechanisms 240 are provided. The two distribution mechanisms 240 are spaced apart in the direction perpendicular to the first line. The first connecting member 226 and the second connecting member 253 are arranged between the two distribution mechanisms. In some embodiments, the first connecting member 226 has teeth arranged along the first line on its opposite side surfaces, and the second connecting member 253 has teeth arranged along the first line on its opposite side surfaces. In other examples, the multiple distribution mechanisms 240 may be spaced apart in other directions.

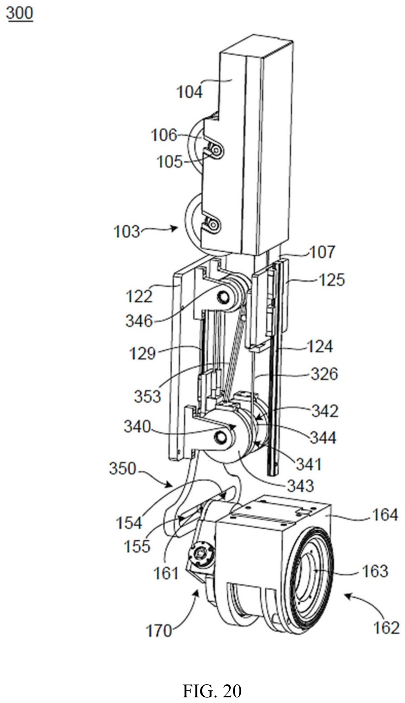

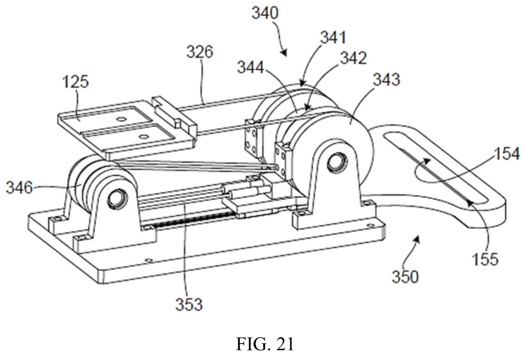

In the third example shown in FIG. 20 and FIG. 21, the distribution mechanism 340 may adopt, as shown in FIG. 7, the third implementation. The distribution mechanism 340 is connected to the first link 120 and rotatable relative to the first link 120 about the third axis which is perpendicular to the first line. The first connecting member is a first flexible transmission member 326, which, for example, may be a cable. The second connecting member is a second flexible transmission member 353, which, for example, may be a cable.

The first flexible transmission member 326 includes a first end 151 and a second end 152 spaced apart in the length direction of the first flexible transmission member 326. The first end 151 of the first flexible transmission member 326 is connected to the force output mechanism 102. In some embodiments, the first end 151 of the first flexible transmission member 326 is fixed to the sliding member 125 on the first link 120, such that the first flexible transmission member 326 is fixed to the force output mechanism 102 via the sliding member 125 and the first guide rail 124. The second end 152 of the first flexible transmission member 326 is fixed to the distribution mechanism 340. The first flexible transmission member 326 is tensioned from its first end 151 to its second end 152. The first flexible transmission member 326 partially rides on the distribution mechanism 340 in a first winding direction.

The second flexible transmission member 353 includes a first end 151 and a second end 152 spaced apart in the length direction of the second flexible transmission member 353. The first end 151 of the second flexible transmission member 353 is fixed to the distribution mechanism 340. The second end 152 of the second flexible transmission member 353 is connected to the pulling mechanism 350. In some embodiments, the second end 152 of the second flexible transmission member 353 is fixed to the pulling mechanism 350, allowing the second flexible transmission member 353 to translate synchronously with the pulling mechanism 350 on the second guide rail 128 along the first line. The second flexible transmission member 353 is tensioned from its first end 151 to its second end 152, and the second flexible transmission member 353 partially rides on the distribution mechanism 340 in a second winding direction. The first winding direction is the same as the second winding direction.

The distribution mechanism 340 may define a first cable groove 341 and a second cable groove 342. The first cable groove 341 circumferentially extends around the third axis to guide the path of the first flexible transmission member 326 in the distribution mechanism 340, and the second cable groove 342 circumferentially extends around the third axis to guide the path of the second flexible transmission member 353 in the distribution mechanism 340. In some embodiments, a part of the first flexible transmission member 326 rides on the distribution mechanism 340 within the first cable groove 341, and a part of the second flexible transmission member 353 rides on the distribution mechanism 340 within the second cable groove 342. The first cable groove 341 and the second cable groove 342 are offset along the third axis to prevent undesired interference between the first flexible transmission member 326 and the second flexible transmission member 353.

The radius of curvature of the part of the first flexible transmission member 326 riding on the distribution mechanism 340 and the radius of curvature of the part of the second flexible transmission member 353 riding on the distribution mechanism 340 may be equal or unequal. The ratio between the two radii of curvature may be adjusted according to moment distribution requirements.

In a plane perpendicular to the third axis, the connecting line between the entry position of the first flexible transmission member 326 into the distribution mechanism 340 and the third axis forms a first connecting line, and the connecting line between the exit position of the second flexible transmission member 353 from the distribution mechanism 340 and the third axis forms a second connecting line. The included angle between the first connecting line and the second connecting line may be either zero or any other value between 0 and 360 degrees. When the included angle is any other value between 0 and 360 degrees, a direction-changing mechanism is needed to redirect the first flexible transmission member 326 and/or the second flexible transmission member 353.

In the third example, the distribution mechanism 340 may include a first pulley 343 and a second pulley 344 that synchronously rotate and are coaxially arranged. The first pulley 343 has the first cable groove 341, and the second pulley 344 has the second cable groove 342. The first flexible transmission member 326 partially rides on the first pulley 343, and the second flexible transmission member 353 partially rides on the second pulley 344. The diameter of the first pulley 343 and the diameter of the second pulley 344 may be equal or unequal. The ratio of diameters between the two pulleys may be selected according to moment distribution requirements.

Further, the first pulley 343 and/or the second pulley 344 is detachably mounted to the first movable member 120, thereby facilitating the replacement of the first pulley 343 and/or the second pulley 344. For example, gears with different diameters may be used to adapt to different moment distribution requirements. In some embodiments, a transmission (e.g., a mechanical continuously variable transmission) may be provided to connect the first pulley 343 and the second pulley 344 to adjust the relative ratio of the working radii of the two pulleys.

In the third example, the direction-changing mechanism 346 has a circumferential surface extending along a fifth axis. The second flexible transmission member 353 partially rides on the direction-changing mechanism 346. The fifth axis may be perpendicular to the first line. Thus, the direction-changing mechanism 346 can reroute the path of the second flexible transmission member 353, allowing more flexible connection and positional arrangements between the distribution mechanism 340 and the pulling mechanism 350. In some embodiments, the direction-changing mechanism 346 may include a third pulley 346 connected to the first link 120 and rotatable relative to the first link 120 about the fifth axis, and the second flexible transmission member 353 partially rides on the third pulley 346.

To enhance the stability of the connection among the distribution mechanism 340, the pulling mechanism 350, and the force output mechanism 102, multiple distribution mechanisms 340 may be provided on the first link 120. Accordingly, the number of the first flexible transmission members 326 and second flexible transmission members 353 may be adjusted correspondingly. In the third example, two distribution mechanisms 340, two first flexible transmission members 326, and two second flexible transmission members 353 are provided. The two distribution mechanisms 340 are spaced apart along the third axis. In the direction parallel to the third axis, the two second flexible transmission members 353 are arranged between the two first flexible transmission members 326; or the two first flexible transmission members 326 are arranged between the two second flexible transmission members 353.

In certain scenarios, the robotic arm has a distal end connected to an instrument-carrying arm. For example, the instrument-carrying arm may be directly connected to the rotating portion of the revolute joint, or connected to the rotating portion via another link and joint. At least one surgical instrument or endoscopic imaging lens is mounted on the instrument-carrying arm and movable relative to the instrument-carrying arm. Before or during surgery, it may need to replace, add, or remove the instruments mounted on the instrument-carrying arm according to surgical requirements, resulting in changes in the gravitational load connected to the rotating portion. During surgery, the surgical instrument or the endoscopic imaging lens may perform actions such as insertion, leading to a shift in the center of gravity of the load connected to the rotating portion. Both situations cause changes in the gravitational moment relative to the first axis. In other words, the gravitational moment that needs to be counteracted changes.

To automatically adapt to such changes, the counterbalance device of the robotic arm may further include a moment arm adjustment mechanism 170. Referring to FIG. 11 to FIG. 12, the moment arm adjustment mechanism 170 adjusts the relative position between the connecting component 161 and the rotating member 163 to adapt to changes in the gravitational moment. In the embodiments, the driving member 171 is configured as a rotary motor having an output shaft 172. The transmission member 173 includes a first gear portion 174, a second gear portion 175, a worm portion 176, and a worm gear portion 177. The first gear portion 174 is fitted on the output shaft 172. The second gear portion 175 is meshed with the first gear portion 174. The worm portion 176 rotates coaxially with the second gear portion 175. The worm gear portion 177 is meshed with the worm portion 176 and rotatable about a fourth axis. The output shaft 172 of the rotary motor is rotatable about an axis perpendicular to the fourth axis. The connecting component 161 is disposed on the worm gear portion 177 and offset from the rotation axis of the worm gear portion 177. Consequently, the driving member 171 moves the connecting component 161 along an arc path around the fourth axis.

The moment arm adjustment mechanism 170 further includes a mounting housing 178 connected to the rotating member 163. In some embodiments, the mounting housing 178 may be fixed to the rotating member 163 by means of screws. The transmission member 173 is disposed inside the mounting housing 178. The driving member 171 is disposed at least partially outside the mounting housing 178, with its drive shaft extending into the mounting housing 178. The second gear portion 175 and the worm gear portion 177 are mounted to two mutually perpendicular sides of the mounting housing 178 and are rotatable relative to the mounting housing 178, such that the rotational axis of the second gear portion 175 is perpendicular to the rotational axis of the worm gear portion 177. Further, the mounting housing 178 defines a rotation slot 179 located concentrically around the rotational axis of the worm gear portion 177. The connecting component 161 passes through the rotation slot 179, thereby being positioned outside the mounting housing 178.

The structure of the robotic arm is described in detail with reference to the embodiments illustrated in FIG. 9 to FIG. 21. However, based on the inventive concept of the present disclosure, the structures of various components in the robotic arm may also be subject to multiple modifications. Reference may be made to the preceding descriptions concerning the counterbalance device, the force output mechanism, the pulling mechanism, the distribution mechanism, and the moment arm adjustment mechanism.

Unless otherwise defined, the technical and scientific terms used herein have the same meanings as those commonly understood by those skilled in the art. The terms used herein are only for the purpose of describing the specific implementation and are not intended to limit the present disclosure. The features described in one embodiment may be applied separately or in combination with other features in another embodiment, unless such features are not applicable in that other embodiment or otherwise specified.

The counterbalance device and the counterbalance system provided in the embodiments of the present disclosure have been described in detail above. Specific examples are used herein to illustrate the principles and embodiments of the present disclosure. The description of the foregoing embodiments is solely intended to facilitate understanding of the core concepts of the present disclosure. The specific embodiments and the scope of application may be varied without departing from the spirit of the present disclosure. In summary, the content of the present disclosure shall not be construed as limiting the present disclosure.

## Claims

1. A counterbalance device for an apparatus with composite joints, the apparatus comprising a frame, a first movable member supported by the frame, and a second movable member connected to the first movable member, the first movable member being translatable relative to the frame along a first line, the second movable member being rotatable relative to the first movable member about a first axis perpendicular to the first line; and
the counterbalance device comprising:
a force output mechanism connected to the frame and configured to provide an output force;
a distribution mechanism connected to the force output mechanism, to allow the force output mechanism to apply the output force to the distribution mechanism at a first load-bearing position of the distribution mechanism; and
a pulling mechanism connected to the distribution mechanism, to allow the pulling mechanism to act on the distribution mechanism at a second load-bearing position of the distribution mechanism, the pulling mechanism being connected to the first movable member and translatable relative to the first movable member along the first line, the pulling mechanism being further connected to the second movable member, and the pulling mechanism being translatable along the first line with rotation of the second movable member about the first axis.

2. The counterbalance device of claim 1, wherein,
the distribution mechanism is mounted to the first movable member and rotatable relative to the first movable member about a third axis perpendicular to the first line.

3. The counterbalance device of claim 2, wherein,
a distance between the first load-bearing position and the third axis is adjustable; and/or
a distance between the second load-bearing position and the third axis is adjustable.

4. The counterbalance device of claim 2 or 3, wherein,
within a plane perpendicular to the third axis, a projection of the first load-bearing position, a projection of the second load-bearing position, and a projection of the third axis lie on a same line.

5. The counterbalance device of any one of claims 2 to 4, wherein the counterbalance device further comprises:
a first connecting member and a second connecting member; wherein,
the force output mechanism acts at the first load-bearing position via the first connecting member, and the pulling mechanism acts at the second load-bearing position via the second connecting member.

6. The counterbalance device of claim 5, wherein,
the distribution mechanism comprises an end connected to the first movable member and rotatable relative to the first movable member, to allow the distribution mechanism to rotate relative to the first movable member about the third axis.

7. The counterbalance device of claim 6, wherein,
the first connecting member comprises a first load-bearing member and a first load-applying member; wherein,
the first load-bearing member is connected to an output end of the force output mechanism, the first load-applying member is connected to the first load-bearing position of the distribution mechanism, the first load-bearing member is provided with a first load-bearing surface, and the first load-applying member acts on the first load-bearing surface and is translatable relative to the first load-bearing surface along a fourth line that is transverse to the first line.

8. The counterbalance device of claim 6 or 7, wherein,
the second connecting member comprises a second load-bearing member and a second load-applying member; wherein,
the second load-bearing member is connected to the second load-bearing position of the distribution mechanism, the second load-applying member is connected to the pulling mechanism, the second load-applying member is provided with a load-applying surface, the load-applying surface acts on the second load-bearing member, and the second load-bearing member is translatable relative to the load-applying surface along a fifth line that is transverse to the first line.

9. The counterbalance device of claim 5, wherein,
the distribution mechanism comprises a first peripheral member and a second peripheral member that extend circumferentially about the third axis and offset along the third axis; wherein,
the first load-bearing position is located on the first peripheral member, and the second load-bearing position is located on the second peripheral member.

10. The counterbalance device of claim 9, wherein,
the first peripheral member comprises teeth arranged circumferentially, the first connecting member comprises teeth arranged along the first line, and the first connecting member meshes with the first peripheral member;
and/or
the second peripheral member comprises teeth arranged circumferentially, the second connecting member comprises teeth arranged along the first line, and the second connecting member meshes with the second peripheral member.

11. The counterbalance device of claim 9, wherein,
the first peripheral member comprises a first cable groove extending circumferentially, the first connecting member comprises a first cable, and the first cable rides on the distribution mechanism along the first cable groove;
and/or
the second peripheral member comprises a second cable groove extending circumferentially, the second connecting member comprises a second cable, and the second cable rides on the distribution mechanism along the second cable groove.

12. The counterbalance device of any one of claims 1 to 11, wherein,
the pulling mechanism is configured to act on the second movable member at a first position of the second movable member, the first position has a same displacement as the pulling mechanism along the first line, and the first position is further translatable relative to the pulling mechanism along a second line that is transverse to the first line and perpendicular to the first axis.

13. The counterbalance device of any one of claims 1 to 11, wherein,
the pulling mechanism is configured to act on the second movable member via at least a connecting component, and the connecting component is translatable relative to the pulling mechanism along a second line that is transverse to the first line and perpendicular to the first axis.

14. The counterbalance device of claim 13, wherein,
the pulling mechanism is provided with a second load-bearing surface extending along the second line, and the connecting component is pressed against the second load-bearing surface and movable on the second load-bearing surface.

15. The counterbalance device of claim 13, wherein,
the connecting component comprises two opposite ends along the first line, wherein one end of the two opposite ends acts on the second movable member, and the other end of the two opposite ends acts on the pulling mechanism.

16. The counterbalance device of claim 13, wherein the counterbalance device further comprises:
a moment arm adjustment mechanism and the connecting component, wherein the moment arm adjustment mechanism comprises a driving member configured to directly or indirectly move the connecting component relative to the second movable member, to change a distance between the connecting component and the first axis.

17. The counterbalance device of claim 16, wherein,
the connecting component is movable in a path lying on a plane perpendicular to the first axis.

18. The counterbalance device of claim 17, wherein,
the path of the connecting component is arc-shaped or straight.

19. The counterbalance device of any one of claims 16 to 18, wherein,
the moment arm adjustment mechanism further comprises a transmission member connecting the driving member and the connecting component.

20. The counterbalance device of claim 19, wherein,
the transmission member comprises at least one of gear transmission, worm gear transmission, pulley transmission, and screw transmission.

21. The counterbalance device of any one of claims 1 to 20, wherein,
the force output mechanism comprises an elastic component configured to provide a constant elastic force; wherein the elastic component comprises an end connected to the frame and another end connected to the pulling mechanism.

22. The counterbalance device of claim 21, wherein,
the elastic component comprises at least one of a constant-force spring and an air spring.

23. A counterbalance system, comprising:
a frame;
a first movable member supported by the frame and translatable relative to the frame along a first line;
a second movable member connected to the first movable member and rotatable relative to the first movable member about a first axis;
the counterbalance device of any one of claims 1 to 22; and
a connecting component connecting the second movable member and the pulling mechanism of the counterbalance device.

24. A counterbalance device for counterbalancing an eccentric mass on a rotating arm supported by a frame, the rotating arm being rotatable relative to the frame about a first axis, and the counterbalance device comprising:
a force output mechanism connected to the frame and configured to provide an output force; and
a pulling mechanism connected to an output end of the force output mechanism, to allow the force output mechanism to apply the output force to the pulling mechanism, the pulling mechanism being connected to the frame and translatable relative to the frame along a first line perpendicular to the first axis, the pulling mechanism being further connected to the rotating arm, and the pulling mechanism being translatable along the first line with rotation of the rotating arm about the first axis.

25. The counterbalance device of claim 24, wherein the pulling mechanism is configured to act on the rotating arm at a first position of the rotating arm, the first position has a same displacement as the pulling mechanism along the first line, and the first position is further translatable relative to the pulling mechanism along a second line that is transverse to the first line and perpendicular to the first axis.

26. The counterbalance device of claim 24 or 25, wherein the pulling mechanism is configured to act on the rotating arm via at least a connecting component, the connecting component is translatable relative to the pulling mechanism along a second line that is transverse to the first line and perpendicular to the first axis.

27. The counterbalance device of claim 26, wherein the connecting component is mounted to the rotating arm and rotatable relative to the rotating arm about a second axis that passes through the connecting component and is parallel to the first axis.

28. The counterbalance device of claim 26 or 27, wherein the pulling mechanism is provided with a second load-bearing surface extending along the second line, and the connecting component acts on the second load-bearing surface and is movable on the second load-bearing surface.

29. The counterbalance device of claim 28, wherein the connecting component comprises at least one roller configured to roll on the second load-bearing surface, and the at least one roller is rotatable about an axis parallel to the first axis.

30. The counterbalance device of any one of claims 26 to 29, wherein the counterbalance device further comprises:
a moment arm adjustment mechanism and the connecting component; wherein,
the moment arm adjustment mechanism comprises a driving member configured to directly or indirectly move the connecting component relative to the rotating arm, to change a distance between the connecting component and the first axis.

31. A counterbalance system, comprising:
a frame;
a rotating arm supported by the frame and rotatable relative to the frame about a first axis;
the counterbalance device of any one of claims 23 to 29; and
a connecting component connecting the rotating arm and the pulling mechanism of the counterbalance device.
